# EUROPEAN PATENT SPECIFICATION

(11) **EP 0 408 223 B1**
(45) Date of publication and mention of the grant of the patent: **08.03.1995**
(21) Application number: 90307139.7
(22) Date of filing: 29.06.1990
(51) Int. Cl.: B01D 61/18, G01N 33/558, G01N 33/48

(54) **Device and method for separation of plasma from blood and determination of blood analytes**
Vorrichtung und Verfahren für die Trennung von Plasma aus Blut und die Bestimmung von Blutbestandteilen
Dispositif et procédé pour la séparation du plasma du sang et la détermination de constituants du sang

(30) Priority: 12.07.1989 US 379009
(43) Date of publication of application: 16.01.1991
(73) Proprietor: Lifestream Diagnostics, Inc., Sandpoint, Idaho 83864 (US)
(72) Inventor: Thakore, Yatin B., East Brunswick, New Jersey 08816 (US); Swanson, Karen L., Hamilton, New Jersey 08610 (US)
(74) Representative: Ablewhite, Alan James

(56) References cited:
- EP-A- 0 269 240
- EP-A- 0 271 854
- EP-A- 0 285 993
- US-A- 4 615 983

## Description

The present invention is directed to a device and a method for separating plasma from blood and determining one or more analytes. More specifically, the present invention is directed to plasma separation from blood and analytical testing of the plasma to measure analytes such as cholesterol, HDL cholesterol, triglycerides, etc.

In many applications such as therapeutics, plasma/serum standards, and particularly for blood analyte determination, there is often a need to separate cells from whole blood. For rapid determination of analytes in serum or plasma, colorimetric methods are quite popular wherein the analyte or a product obtained by conversion of this analyte, reacts with a chromogen to give color, the intensity of which is proportional to the concentration of the analyte. When using whole blood for determination of an analyte, it is essential to remove the red cells from the blood so that the color of the reaction is not masked by the color of the blood. Centrifugation of the blood or the clotting of blood are two common prior art methods used to isolate plasma or serum from whole blood. However, these result in an additional sample preparation step. It would appear that since red blood cells are rather large (about 8 micrometers in diameter), one could conceivably use a microfilter of a smaller pore size to obtain clear plasma. However, this does not usually work for the following reasons:
(a) When one puts a drop of blood on a dry hydrophilic membrane with pore sizes in the 3 to 8 micrometers range, the blood gets absorbed into the pores and onto the microfibrils of the filter. Often due to this rapid absorption, hemolysis of blood occurs wherein the fragile red blood cells rupture and the broken cells and/or hemoglobin from the cells leak through. As a result, generally, the plasma coming through is not clear but reddish and the throughput may also be small because the filter may clog up rapidly due to the high solids content of the blood (typically 50% of blood volume is cells). To compound the problem, blood may start clotting in the membrane further promoting the clogging. Due to this clogging, very often large analytes such as lipoproteins may be held back and whatever passes through the membrane may be significantly diluted with respect to these large analytes. The concentration of these analytes in the filtrate may therefore be significantly lower than in plasma or serum and may vary with both applied sample volume as well as with blood hematocrit (solid content), hence yielding unreliable results.
(b) When one places a drop of blood onto a hydrophilic microporous membrane with pore sizes of less than 3 micrometers, the clogging on top of the membrane appears to be especially rapid and usually neglibible plasma gets through. Again the same problems as mentioned above with clogging are encountered.
(c) Using a depth filter in addition to or in place of the microfilter may likewise be problematic. Due to higher hold up and absorptive capacity of the depth filter, volume requirements of blood become rather excessive and the problems of hemolysis and clogging still exist. An exception to this is the depth filter made up of glass fibers which was shown to separate plasma/serum from blood rather effectively. This method is described in US-A-4,477,575. However, many of the problems of other "dry" membrane systems remain, although to a lesser degree.

In order to overcome the problems of dry membrane test systems, numerous wet or semi-wet microporous membrane test systems have been proposed. Thus, when wetted with water or with aqueous solutions, the microfibers of these hydrophilic membranes are hydrated by a thin layer of water. This significantly reduces the absorptive capacity of the membrane. It is possible to get fairly clean plasma across this wetted microporous membrane of pore sizes of about 5 micrometers or less in this fashion. However, wet and semi-wet systems have three major drawbacks. First, wet and semi-wet systems may involve wetting the membrane prior to use and this adds an additional step for a diagnostic test. Second, when dealing with small volumes of a few microliters, as are encountered in diagnostic tests, this would dilute the plasma and the extent of dilution would depend upon the volume of sample. Finally, in a wet membrane, the rate of transport of plasma through the membrane will be governed by diffusion rather than capillary pull and may take an excessively long time thereby affecting the overall response time of a diagnostic test.

This approach of using a wet microporous membrane for separation of plasma from whole blood is indeed being used very successfully in a process called plasmaphoresis. In this process, the microporous membrane is first wetted with isotonic saline in a step which is called priming the membrane. Blood is then pumped through one side of the membrane and a transmembrane pressure differential is maintained. Because of the externally applied pressure difference, the process is fairly rapid. Additionally, a relatively large amount of blood is processed (on the order of a liter) and hence dilution due to isotonic saline in the membrane is negligible. Therefore, even though plasmaphoresis is an effective way of obtaining plasma from blood on a process scale, it is not very practical for a simple and rapid diagnostic test requiring on-line serum/plasma separation for microliter quantities, without external pressures and preferably without dilution.

Glucose reagent strips were among the first to utilize whole blood for blood glucose measurements. These colorimetric tests for whole blood based on dry-strip technology became quite popular due to the possibility to obtain rapid analysis of glucose from whole blood without sample pre-treatment. These early tests relied on diffusion of glucose from whole blood through thin, transparent and tightly structured membranes (U.S.-A-3,092,465; 3,298,789; 4,543,338). A drop of blood is placed on the test-pad and after a pre-determined period of time the blood is either washed off or wiped off and the color developed underneath is then compared to a color chart or read in a reflectance meter. These tests do not require a precise metering of the blood but do require a blood wash-off or a wipe-off step and precise timing for the same. While this approach is satisfactory for a relatively small hydrophilic molecule such as glucose which can diffuse rather easily through a thin tightly structured membrane, it is not feasible to measure large analytes such as proteins or hydrophobic analytes such as cholesterol or triglycerides which are complexed with proteins and are carried in the blood stream as part of lipoproteins ranging in size from a few hundred thousand to several million in molecular weights. These relatively large molecules cannot penetrate the dense membranes used in the glucose strips and attempts to separate plasma or serum from whole blood with more open membranes and without pressure or external influences were not successful due to reasons mentioned above.

The discovery that certain depth filters made up of glass fibers could successfully separate red cells from whole blood (U.S.-A-4,477,575) paved the way for successful commercialization of an instrument called "Reflotron" by Boehringer Mannheim Diagnostics (Trademark) where the separation of clear serum or plasma occurs within the dry strip itself without need of an external force such as pressure or centrifugation. This made analysis of various analytes possible from whole blood in a simple inexpensive manner. However, although blood analysis with Reflotron is convenient, a metered amount of blood must be placed onto the reagent pads and there is a need to crush the pad to bring all the reagents into intimate contact with the separated serum/plasma. Additionally, it can perform only one reaction at a time and typically the chemistries take about three minutes each.

Thus, while many new developments have occurred and have been patented, the need for a convenient, efficient, easy-to-use, reliable test method and device for testing one or more analytes from whole blood plasma has not been satisfied until the development of the present invention.

The present invention involves a method and device for obtaining plasma from whole blood and determining one or more analytes contained therein. A sample of blood is first applied to a physical transport medium which moves at least a portion of the blood from a remote location thereon to a second location thereon to a first surface of a microporous plasma separation membrane. This tangential movement is achieved by gravity, absorption and/or capillary action. The physical transport medium may be a sheet of fabric with weave or otherwise or a channeled or grooved synthetic material. Once the blood has moved to the first surface of the microporous plasma separation membrane, the plasma is absorbed and passes through the membrane, thereby separated from the whole blood (and the red color). The plasma reaches the second (opposite) surface of the microporous plasma separation membrane and contacts a plasma collecting test membrane. This test membrane is capable of reacting with the plasma (i.e. one of its components) to display at least one analyte characteristic thereof. Preferably, colorimetric reaction takes place and color intensity indicates concentration level of the analyte in the blood.

According to the present invention there is provided a device for obtaining plasma from whole blood and determining blood analytes thereof, which comprises:
(a) a microporous plasma separation membrane having a first surface and a second surface;
(b) a physical transport medium contacted in part with said first surface of said microporous plasma separation membrane which medium is capable of moving at least a portion of blood to be tested from a remote location on said physical transport medium to said first surface of said microporous plasma separation membrane; and
(c) a plasma collecting test membrane in contact with the second surface of said microporous plasma separation membrane for receiving said separated plasma, said test membrane being capable of reacting with said plasma to display at least one analyte characteristic thereof; characterised in that said microporous plasma separation membrane is a skinned membrane, in which one or both surfaces of the skinned membrane have smaller and uniform pore-sizes, as compared to the middle region of the skinned membrane.

According to the present invention there is furthermore provided a method of obtaining plasma from whole blood and determining blood analytes thereof, as claimed in claim 17.

The present invention described herein is more fully appreciated and understood when the disclosure is taken in conjunction with the appended drawings,
wherein:
Figure 1 illustrates a photographic representation of a 5 micrometer nitrocellulose plasma separation membrane used in the method and device of the present invention;
Figure 2 shows a photographic representation of a 3 micrometer cellulose plasma separation membrane used in the present invention;
Figure 3 shows a side cut view of a device according to the present invention which includes a support substrate, a lateral physical transport medium, a microporous plasma separation membrane and a plasma collecting test membrane;
Figure 4 shows a top diagramatic view of the device shown in Figure 3;
Figure 5 shows an alternative embodiment of a device according to the present invention utilizing a channeled substrate as the physical transport medium;
Figure 6 shows another alternative embodiment of a device according to the present invention wherein the physical transport medium is a combined channeled substrate and capillary action transport membrane;
Figure 7 illustrates the top view of an alternative device according to the present invention;
Figure 8 illustrates a side view of a device according to the present invention having multiple plasma collecting test membranes for determining three different analytes; and,
Figure 9 illustrates a device according to the present invention which includes radially arranged plasma collecting test membranes which permit analyte determination for four different analytes.

The present invention is directed to obtaining clear plasma from microliter quantities of whole blood rapidly, efficiently, without causing dilution of blood analytes and without relying on externally applied forces or without any sample pretreatment. It is also directed to the process of separation of plasma in a device for determination of blood analytes. The present invention plasma separation method can be utilized for colorimetric detection of blood analytes. The devices of this invention enable the plasma to come into intimate contact with all the necessary enzymes, proteins or other reagents and chromogens without external pressure or without denaturing blood or sensitive reagents. The test strip device of this invention also enables one to do multiple chemistries on the same strip with a single sample application in a very quick, simple and reliable manner.

Primarily, the present invention relies upon application of blood at a first (remote) location on a physical transport medium and movement of the blood to a second location on the medium for separation of plasma and subsequent testing. Typically, the present invention utilizes tangential flow of blood underneath a suitable microporous plasma separation membrane so that the red cells are retained on the underside but clean plasma is obtained on the top surface of the membrane. Capillary pressure, both within the membrane matrix and by suitable channels and/or a suitable open mesh underneath the microporous membrane, may provide the appropriate driving force for the tangential flow and no external forces are necessary. For filtration of highly viscous liquids or for slurries or suspensions with high solids content, tangential flow across the membrane is much more efficient than the dead-ended filtration. The problems of clogging of the membrane are substantially reduced in tangential filtration mode. In this system, a viscous fluid or suspension is swept across one side of the membrane, and clean filtrate is collected across the other side.

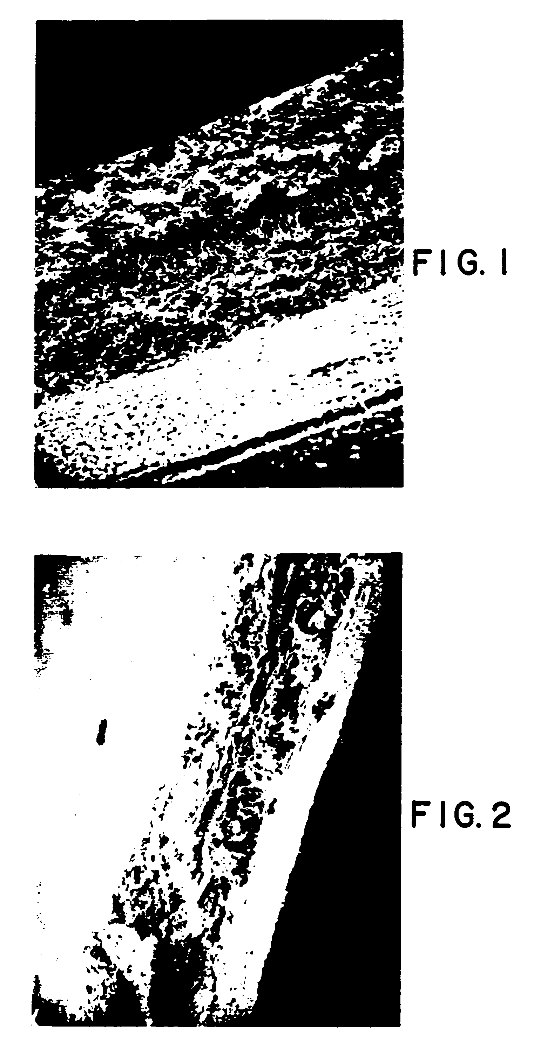

This invention uses this principle, for a diagnostic strip, on a miniature scale. For small volumes of blood which can be obtained by finger-pricking, the driving force for blood flow may be provided entirely by the capillary pull. To do this efficiently, it relies heavily on both the pore-structure of the separation membrane and the physical transport medium and support structure underneath it. Additionally, the choice of the microporous separation membrane becomes even more important than for other conventional applications. An ideal separation membrane should wet easily with serum or plasma, should not allow the red cells to migrate to the top surface, should not cause hemolysis of the red cells, should have a good capillary pull and a fairly uniform pore-structure near the surfaces. Out of several dozen commercial microporous membranes which were screened, three were found to be particularly preferable, a 5 micrometer microporous membrane made from nitrocellulose of Schleicher and Schuell (Keene, New Hampshire) performed optimally in separating plasma from whole blood. In general, suitable pore-sizes of such membranes should be between about 0.02 to 10 micrometers, with the preferred range between 1 to 5 micrometers. Skinned membranes, wherein one or both surfaces of the microporous membranes have much smaller and uniform pore-sizes, as compared to the pore-sizes in the middle region, are found to be particularly suitable. Unlike most commercial microporous membranes which are generally isotropic in their cross-section, these membranes show double-skinned structures which may be the reason for their effectiveness. Figures 1 and 2 show the cross-section of the 5 micrometer nitrocellulose and the 3 micrometer cellulosic membrane respectively.

Other important components of this invention are the physical transport medium and the substrate onto which this microporous separation membrane is placed. Typically, this membrane is placed on a plastic substrate and between this substrate and the membrane is placed a physical transport medium which may be a material with open structure and hydrophilic surface to achieve tangential flow of the blood.
Examples of materials with such open structures are various types of polymeric meshes, cloth, tissue papers, gauze etc. Ideally the hold up volume and the absorptive capacity of these open-structured materials should be very small so as to minimize the loss of blood due to absorption and the surface on a macroscopic scale should be fairly smooth so as to provide good contact with the microporous separation membrane. Woven fabrics from many monofilament yarns such as polyester and nylon are particularly useful. Even though the pore sizes of the transport media are not critical, it would be preferable to have them at least an order of magnitude larger than that of the analyte of interest, so that these materials do not have any sieving properties and so that their sole function is to assist in smooth and rapid flow of blood. As a further aid for rapid movement of blood underneath the microporous separation membrane, channels of well-defined geometry or grooves may be included on the plastic substrate, underneath the open-structured material. Channels 1 to 10 mm wide and 25 to 375 micrometers [1 to 15 mil] deep work quite well, 1 to 4 mm width and 50 to 250 micrometers [2 to 10 mil] depth being the preferred range.

In practice, an open structured material such as a monofilament woven fabric is placed onto the plastic substrate with or without the channels, and the microporous separation membrane is placed on the top such that part of the fabric extends beyond the membrane. A plasma collecting test membrane such as a piece of filter paper or another hydophilic membrane is placed on top of the microporous separation membrane. This will absorb the clean plasma as it comes through the microporous separation membrane and may be loaded with reagents specific for a given analyte. This whole assembly may then be secured by placing another plastic cover on the top. Blood applied to the physical transport medium through an orifice in the plastic cover at a location remote from the separation membrane, migrates along to a location next to (under) the microporous plasma separation membrane, thereby creating a thin film of blood along one surface of the microporous plasma separation membrane. Clean plasma available from the top surface of this membrane saturates the test membrane and if it is loaded with reagents, the analyte reacts with these reagents producing color or other analyte indicator.

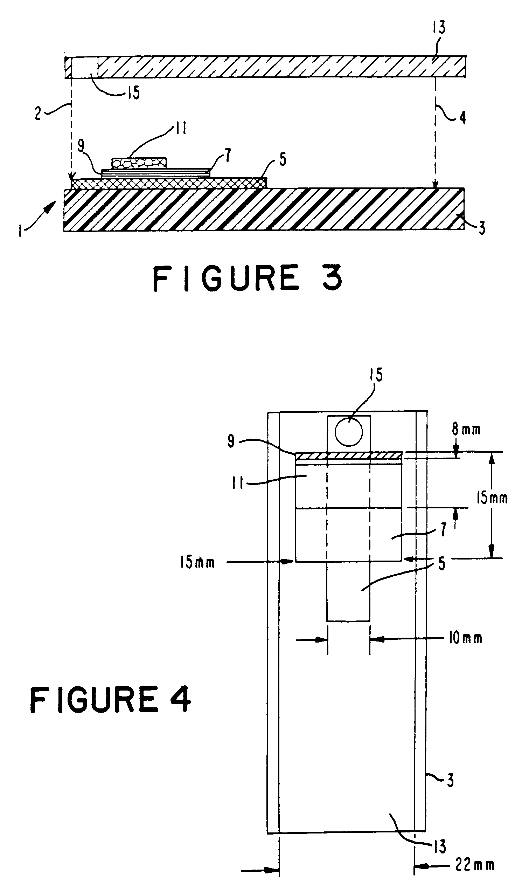

Referring now to Figures 3 and 4, there is shown a side cut view and a top view of a test strip device 1. The device 1 has an inert substrate 3 and a physical transport medium 5 attached thereto. In this embodiment, physical transport medium 5 is a woven cloth material. Atop physical transport medium 5 is microporous plasma separation membrane 7 and optional hydrophobic barrier strip 9, adjacent thereto as shown in both figures. Of less length and placed atop of microporous membrane 7 is plasma collecting test membrane 11. Clear plastic cover 13 with orifice 15 is adhered over the tops of the other components as shown by the arrows 2 and 4 in Figure 3. Top view Figure 4 shows all of the components, at least in part, due to the fact that cover 13 is clear.

As now can be seen, the user simply places a drop of blood in orifice 15 ( a remote location from microporous plasma separation membrane 7 ). The blood flows down physical transport medium 5 and under microporous plasma separation membrane 7 to a first (bottom) surface thereof. Plasma separates and flows upwardly through membrane 7 and is, at this point, essentially clear in color. It migrates to a second (opposite, i.e. top) surface of membrane 7 to plasma collecting test membrane 11. Here the analyte(s) react to establish concentration(s). For example, in this case , test membrane 11 contains reagents specific for the analyte or analytes of interest, e.g. chromogens which reveal colorimetric measurement of cholesterol, glucose or the like. Further, the dimensions shown in Figure 4 are merely exemplory and are not critical.

In general, the essential components of the device are the physical transport medium, the microporous plasma separation membrane, and the plasma collecting test strip. Although not preferred, a microporous plasma separation membrane may be extended and graded to act both as a transport medium and a separation membrane. However, more leeway as to blood volume, dimensions and "overflow" control are achieved when the physical transport medium is one or more components which are elements separate and different from the microporous plasma separation membrane. Likewise, the transport medium may act as a substrate or a separate, inert and inactive substrate may be utilized.

The combination of a separate physical transport medium and a separation membrane is chosen such that when blood is placed at the remote blood application area, it is rapidly pulled underneath the microporous membrane and clean plasma seeps across this membrane and saturates the reactant containing test membrane. Further, the test membrane may be a single layer or may be a compilation e.g., a reagent containing layer as well as a chromogen containing layer which is preferably a porous hydrophilic layer. The enzymes or reagents in these layer(s) dissolve in the plasma and react with the analyte producing color. This type of system is particularly appropriate for determining analytes such as glucose, cholesterol, triglycerides, uric acid, creatinine, alcohol, creatine kinase, cholinesterase, AST, ALT, etc. wherein an enzyme or a series of enzymes convert an analyte to hydrogen peroxide which then reacts with chromogen, contained in the test membrane. Many of the enzymes used in such diagnostic strips are particularly susceptible to denaturation when contacted by many chromogens for an extended period of time. By separating the enzymes and the chromogens in two separate layers and yet keeping them in close proximity, the long term stability of the reagents can be preserved. For analyzing analytes such as albumin or other proteins, amylases, calcium, inorganic phosphorus, etc. wherein the reagents used are not biomolecules such as enzymes or antibodies but are stable chemicals and the color formed is due to selective complexation of the analyte with the reagents, the porous layer can be eliminated and all the reagents can be combined in a single layer membrane. It is also possible to load some of the reagents elsewhere in the system such as in additional layers, although this is not essential, provided that they don't hemolyze the cells or damage them in any way. It is best to view the final color at the top of the test membrane since it also acts as a hiding layer to mask the color of the blood. In such a case, an additional layer, if necessary for chromogens, could be transparent or translucent. In order to avoid the bleedthrough of the color developed during the test downstream from the device onto the test membrane, an optional hydrophobic material or glue can be applied on the exposed edge of the separation membrane just downstream of the physical transport medium (not shown in the Figures). Such a hydrophobic surface barrier prevents the color from diffusing down the separation membrane, but is not always necessary.

In the alternative, when a test membrane having a first reactant (reagent) and second reacting component (chromogen) is used, a single test membrane instead of a plural layer may be used. Thus, in some embodiments, the reagents and chromogens may be contained in dry or semi-wet form or even wet form in the same membrane structure, or one or the other may be encapsulated or otherwise isolated within the same membrane.

Further, the device may be used without any additional sample preparation for detection of certain analytes which normally requires a precipitation step for removal of interfering analytes. The precipitants, for example, can be incorporated within the microporous plasma separation membrane or in the physical transport medium, so that the precipitation step occurs on-line and the plasma reaching the test membrane is free of the interferent. If, due to the nature of the precipitant, it is advisable not to have direct contact of the precipitant with the blood, then an additional layer containing the precipitant can be placed between the separation membrane and the physical transport medium. An application that makes use of the precipitation step is the measurement of high density lipoprotein cholesterol (HDL cholesterol) which typically needs precipitation steps using precipitants such as polyethylene glycols, dextran sulfate, or magnesium tungstate to precipitate out cholesterol in low density and very low density lipoproteins (LDL and VLDL), followed by centrifugation. The present invention device achieves this on-line by loading the precipitant into the microporous plasma separation membrane.

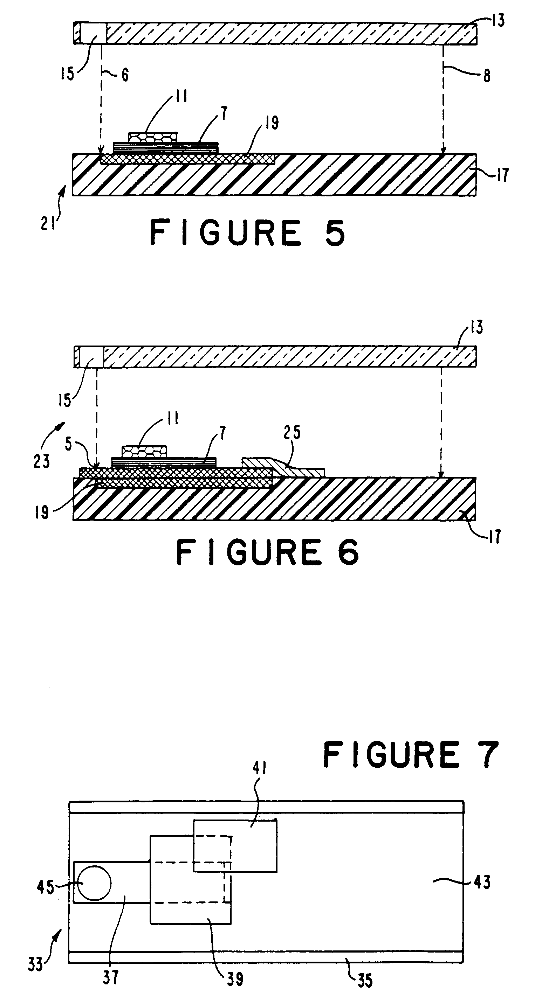

These devices are self-monitoring because a microporous membrane cannot be over-filled or over-saturated by capillary filling. Once the plasma collection membrane is saturated, the excess blood and plasma simply migrate down the transport medium. If, for some reason, the sample volume is so large as to exhaust the absorptive capacity of the transport medium, then the excess blood will merely stay at the application area without getting absorbed. In case the sample volume is not enough, then only part of the test membrane will be saturated and colored. The precise control over the thicknesses (and hence the void volume) of the test membrane is not critical because even if the saturation capacity of this membrane varies with the thickness, the concentration of the analyte and hence the intensity of the final color produced will be independent of the thickness of this membrane. The minimum volume needed to saturate the plasma collecting test membrane with plasma will depend on the thickness of this membrane, the geometry of the system and the hematocrit of the blood: for the system shown in Figures 3 and 4, it is approximately 10-15 microliters. When using plasma or serum in place of whole blood, the volume requirements are half as much. To further minimize the required minimum volume the size of the test membrane and/or the thickness may be decreased as long as the test membrane is sufficiently opaque to mask the color of the blood underneath it. If the test membrane is not of sufficient opacity, then one could use a geometry similar to that shown in Figure 7 where the color developed is not directly over the blood path.

Figure 5 shows a device 21, which is similar to the one shown in Figures 3 and 4 and like parts are like numbered. Note that physical transport medium 19 is now channels or grooves established in substrate 17 itself. Arrows 6 and 8 show assemblage.

Figure 6 likewise shows a device 23 with like parts being identically numbered, and here, both the cloth physical transport medium 5 of the Figure 3 device and the grooved physical transport medium 19 of the Figure 5 device are combined. Additionally, absorbant storage medium 25 rests in part on transport medium 5 and on substrate 17. This optional storage medium may be used to absorb additional (excess) blood to draw the excess past the separation membrane 7. Any blood absorbing material may be used, although this is not an essential feature of the invention.

Figure 7 shows a device 33 which has substrate 35, physical transport medium 37 (e.g. a sheet material such as a gauze or synthetic having the necessary capillary action), located on substrate 35, plasma separation membrane 39 over physical transport medium 37, and plasma collecting test membrane 41 which is partly over the separation membrane 39 and partly located directly on substrate 35, as shown. This arrangement permits the use of a very thin separation membrane which may show through some of the red color. By offsetting part of the test membrane, the isolated portions will show true colorimetric readings without a pinkish background. Top cover 43 with orifice 45 is clear plastic and the user uses this strip in the same manner as described above.

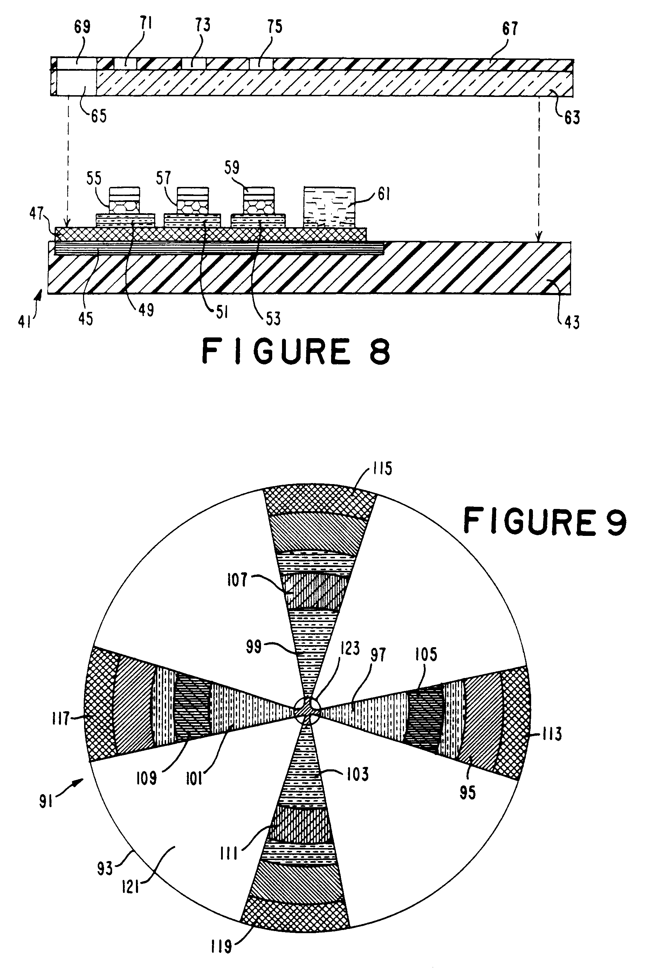

Advantageously, the method and device according to the present invention may be used for a plurality of simultaneous testings. Thus, it is possible to combine various chemistries on the same device. This is particularly advantageous for example in testing on-site in doctor's office, where a patient needs to be checked for several analytes to diagnose a condition. In the strips of this invention several related tests can be combined on the same strip and determination of multiple analytes can be made within a few minutes from application of a single blood sample which can be obtained by finger-prick. Thus, Figure 8 shows one geometry combining a lipid panel (cholesterol, HDL cholesterol and triglycerides) onto a single strip. There is shown in Figure 8 device 41 having substrate 43. The physical transport medium in this embodiment includes channels 45 located on substrate 43 as well as sheet 47 located above channels 45. Three microporous plasma separation membranes 49, 51 and 53 are spaced apart and located on sheet 47 as shown. Membrane 51 additionally contains a precipitant for LDLs and VLDLs (low and very low density cholesterols). Atop each of the separation membranes 49, 51 and 53 are multilayer plasma collecting test membranes 55, 57 and 59 respectively. The plasma collecting test membranes 55, 57 and 59 contain the necessary reactants for three different analytes, in this case cholesterol, HDL cholesterol and triglycerides respectively. Optional absorbant storage medium 61 is also included. The top cover in this embodiment includes a first clear plastic layer 63 with orifice 65 and an opaque layer 67 containing orifice 69 for blood sample transmission and orifices 71, 73 and 75 for visual comparison for colorimetric measurements of the respective analyte reactions at test membranes 55, 57 and 59.

Figure 9 shows a different geometry for obtaining plural analyte characteristics and, in this case, contains four different analyte tests. Thus, device 91 includes substrate 93 and, in a large radially expanding cross, physical transport medium 95 which is placed on top of substrate 93 and extends radially from the center to approximately the outer edge of substrate 93 and under all other components hereafter described. Atop physical transport medium 95 are four separate microporous plasma separation membranes 97, 99, 101 and 103. Atop a portion of each of these are plasma collecting test membranes 105, 107, 109 and 111, respectively, and these in turn contain reactants for colorimetric determination for four different analytes such as glucose, cholesterol, HDL cholesterol and triglycerides. Optional absorbant storage media 113, 115, 117 and 119 are placed on top of the outer ends of physical transport medium 95. Clear plastic cover 121 is placed on top of all the other components and has a single orifice 123 for blood sample application in the center as shown. This device 91 permits nearly simultaneous testing of four different analytes by application of a single blood drop.

The following examples further support the effectiveness of the present invention:

### Example 1

A device such as that shown in Figures 3 and 4 was prepared except that tissue paper is used as the the physical transport medium to facilitate the flow of blood underneath a 5 micrometer pore-size nitrocellulose separation membrane (Type AE 98, Schleicher and Schuell), a microphotograph of which is shown as Figure 1. The plasma collecting test membrane in this case was a 0.45 micrometer nylon 6,6 membrane. It is advantageous to use for the test membrane a microporous membrane of a pore size smaller than the plasma separation membrane for plasma collection; it will then also act as a secondary membrane in case the primary membrane has some defects. A drop of blood (20-40 microliters in volume) is placed at the blood application area and within about 1 to 2 minutes the plasma collection membrane gets saturated with clear plasma.

### Example 2

A Figure 5 type of device was constructed wherein the bottom support plastic has a 3 mm wide and 125 micrometers [5 mil] deep channel built in. No open structured material is used as a blood transport material. To prevent the blood from going over the plasma separation membrane, a hydrophobic tape is put on the top surface of this membrane. The plasma separation membrane is the very same as in Example 1. When a drop of blood is placed on the blood application area, clear plasma saturates the plasma collecting test membrane in about 1 minute.

### Example 3

A Figure 6 device with a geometry utilizing both the channels and an open mesh was made. The channel geometry is the same as in Example 2 but a woven fabric from monofilament polyester is used to facilitate the transport of blood. The plasma separation membrane is of the same type as in above examples. When a drop of blood is placed on the blood application area, clear plasma saturates the plasma collection membrane in about 10 seconds.

### Example 4

The same geometry as shown in Example 3 is used except that the plasma separation membrane is 8 micrometer nitrocellulose (Type AE99, Schleicher and Shuell). The plasma collecting test membrane is saturated in about 10 seconds.

### Example 5

The same geometry as in Example 4 is used except that cellulosic membrane (Micron Separations, Inc.) is used for the separation membrane. A microphotograph of this cellulosic membrane is shown in Figure 2. The plasma collecting membrane is saturated within 30 seconds.

### Example 6

In the geometry of Example 3, an additional filter paper is introduced to serve as an absorption pad to soak up excess blood as shown in Figure 7. The rest of the components are the same as in Example 3. Blood samples from about 15 to 100 microliters are placed in the blood application area and the plasma collection membrane is saturated in about 10 seconds.

### Examples 7-15

Examples 7 through 15 are illustrative of the geometry, assembly and functioning of the devices. In these illustrative examples, a 5 micrometer nitrocellulose membrane (Schleicher and Schuell) was used as the plasma separation membrane (#1), 0.45 micrometer pore-size nylon 6.6 membrane (Micron Separation Inc., Westoro, MA) as the plasma collecting test membrane and a woven polyester mesh as the physical transport medium to facilitate the transport of blood, lens tissue-paper or a dialysis membrane of 1.66 . 10⁻²³ kg (10,000 dalton) cut-off (Spectrum Medical Industries,Inc.,L.A.,CA) was used to load the chromagen, white PVC or polystyrene plastic (1.30-0.71 mm sieve opening [15-25 gauge]) was used as the substrate and a clear PVC or vinyl plastic (2.38-1.68 mm sieve opening [8-12 gauge]) was used as the top cover. Glues such as polybutene or silastic or PVC based, or medical grade double-stick tape, all of which are relatively inert toward the chemistries, where used in these devices to provide either a hydrophobic barrier or a good seal. The chromogen used in conjunction with the various chemistries involving enzymes as the reagents is 3,3', 5,5' tetramethylbenzidine dihydrochloride (TMBD) which in the presence of horseradish peroxidase gives bluish green color when it reacts with hydrogen peroxide generated from the reactions of the analyte. TMBD can be substituted by a number of other chromogens such as phenol or its derivatives and 4 amino antipyrine, or a number of leuco dyes, e.g. from arylmethine family (e.g. leuco malachite green) or by solubilized reactive dyes in their leuco form. The porous lower layer of the test membrane was soaked into the solution of the chromogen and allowed to dry. Typically this solution consisted of 0.3g TMBD, 0.5g of sodium cholate, and 0.5-2g of polyethylene glycol (PEG) (molecular weight 1.328 . 10⁻²³ kg (8000 daltons)) dissolved in 20 ml of reagent alcohol and 0.1M pH 3 phosphate buffer (3:1 by vol). The top layer of the test membrane was tissue paper soaked in the chromogen solution and upon drying stays tacky due to a combination of sodium cholate and PEG. This tackiness enables it to have an intimate contact with the reagent lower layer. To load the reagents, reagent solutions in appropriate buffers are made at the desired concentrations and the membrane is either soaked into this solution or the solutions pipetted onto the membrane till it is saturated. The membrane is then allowed to dry at room temperature. Typically the reagent membrane needs 10 microliter of reagent solution per square cm area for saturation. The solutions used for the various tests illustrated in the examples below are as follows: (a) cholesterol: 400 units of pancreatic cholesterol esterase, 40 units of cholesterol oxidase from Nocardia, and 200 units of horseradish peroxidase per milliliter of 0.1M pH 7 phosphate buffer. (b) Triglycerides: 2000 units of lipase from chromobacterium viscosum, 300 units of glycerol kinase from arthrobacterium sp., 300 units of glycerol phosphate oxidase (Microbial), and 500 units of horseradish peroxidase per milliliter of 0.1M phosphate buffer at pH 8. Additionally adenosine triphosphate (ATP) solution is prepared at a concentration of .625g/ml and is combined with the above solution in the ratio of 4 parts of enzyme solution to 1 parts of ATP solution. (c) glucose: 80 units of glucose oxidase from aspergillus niger and 300 units of horseradish peroxidase per milliliter of 0.1M phosphate buffer at pH 7.

### Example 7

Test strips for measurement of total cholesterol were made as per Figure 6 where the reagent membrane contained the enzymes for cholesterol as described above. Blood or serum samples ranging in volume from 10 to 40 microliter were placed at the blood application area. Typically it took about 10 to 15 seconds to saturate the test membrane and in about 30 seconds the color was fully developed which was then read in a reflectance meter or compared to a color chart.

### Example 8

Test strips of cholesterol were made as per the geometry of Figure 6 and with the reagent composition as described above. Samples of whole blood ranging in volume from 15 to 50 microliters were applied at the blood application area. The color development typically was complete in about 30 to 45 seconds.

### Example 9

HDL cholesterol strips were made as per geometry of Figure 6. The only difference between the cholesterol strips of Example 7 and the HDL strips is that the plasma separation membrane 1 in this case was first soaked in an aqueous 20% by weight solution of PEG 2000 ( a precipitating agent for LDL and VLDL cholesterol) and allowed to dry prior to use. 10 to 40 microliter samples (whole blood, serum or plasma) were applied at the sample application area and color development was complete in about 1 minute.

### Example 10

Triglyceride strips were made as per the geometry of Figure 6 and the test membrane was loaded with the enzymes as described above. 10 to 40 microliter samples (whole blood, serum or plasma) were applied at the sample application area and color development was complete in about 1 minute.

### Example 11

Glucose test strips were made as per the geometry of Figure 6 wherein the test membrane was loaded with the enzymes as described above. 10 to 40 microliter samples (whole blood, serum or plasma) were applied at the sample application area and color development was complete in about 30 seconds.

### Example 12

Test strips for the lipid measurement were prepared as shown in Figure 8. The three test membranes for individual geometries were as described above and the separation membrane for HDL cholesterol was treated with PEG as in Example 9. 40 to 100 microliter samples (whole blood, serum or plasma) were applied at the sample application area and color development was complete in about 3 minutes.

### Example 13

Devices for simultaneous measurement of cholesterol (total and HDL), glucose and triglycerides was prepared as per the geometry of Figure 9. 50 to 100 microliter samples (whole blood, serum or plasma) were applied at the sample application area and color development was complete in about 2 minutes.

### Example 14

Devices for the measurement of total cholesterol and HDL cholesterol were made. 20 to 80 microliter samples were applied at the sample application area and the color development was complete in about 1 minute.

### Examples 15-21

In the following Examples 15 - 21, the enzymes and chromogens were loaded in a single layer plasma collecting test membrane. The chromogen was 3,3′, 5,5′ tetramethyl benzidine which was dissolved at a concentration of .03 g/ml in acetone or at about .02 g/ml in a 3% solution of phenol in toluene. The enzymes for cholesterol were loaded from their aqueous solutions as described above and the test membranes were allowed to dry. After drying, the test membranes were loaded with chromogen solution ( either toluene or acetone based ) and again allowed to dry. The geometry used in testing was as shown in Figure 7, except that the separate chromogen layer was eliminated and additionally an aperture was provided on the top plastic cover over the plasma collecting membrane to allow for oxygen, since the reactions seemed more sensitive to oxygen requirements than when the chromogen was loaded separately in another matrix. For aesthetic purposes and to protect the reagent area from outside contamination, an air-permeable membrane, such as a thin (∼20 micrometer thick), transparent polyurethane membrane coated on one side with porous adhesive (e.g. from Acutek, Inglewood, California) can be placed over the aperture. Such films were found to allow adequate transport of oxygen to the reaction zone.

### Example 15

A nylon -6,6 membrane of 0.45 micrometer pore-size was loaded with cholesterol enzymes and chromogen from acetone solution. When blood or serum samples in volumes ranging from 15 to 50 microliters were applied at the blood application zone, in a Figure 7 type device, color developed in about 30 seconds and colors were accurately proportional to the cholesterol content.

### Example 16

The same test was done as in Example 16 with a cellulosic test membrane of 0.2 micrometer pore-size. The chromogen, however, was loaded from its solution in toluene. Again similar results were obtained.

### Example 17

The test of Example 15 was repeated. This time, however, after loading and drying with cholesterol enzymes, the test membranes were saturated with acetone, and after partial evaporation of acetone, a mixture containing 90:10 parts by volume of toluene and dodecane was added. After a few minutes of drying the test membrane, chromogen in toluene solution was applied. When the test membrane dries, the non-volatile dodecane component is left behind. These membranes are, therefore, not quite dry but contain the hydrophobic, non-polar dodecane and can be considered pseudo-liquid membranes. Non-polar liquids such as dodecane create a hydrophobic environment around the enzymes and give them added stability against denaturation and expecially under humid environment. When tested, the performance was similar to above.

### Example 18

The test of Example 15 was repeated but this time with a test membrane from HYPAN® multiblock polymer (Kingston Technology , Dayton, N.J.) and described in U.S.-A-4,331,783; 4,337,327; 4,369,294; 4,379,874; 4,420,589 and 4,370,451. This test membrane was made over a polyester cloth backing and contained approximately 5-10 mole % of acrylamide groups and the remainder were acrylonitrite groups. The structure of the test membrane was asymmetric with the pores on the more open side of being larger than 1 micrometer. When this membrane was tested as in Example 15, similar results were obtained.

### Example 19

The same test as that in Example 17 was repeated with HYPAN membrane of Example 18. HYPAN membrane in this pseudo-liquid state showed similar behavior.

### Example 20

The test of Example 15 was repeated with a microporous HYPAN membrane which was symmetric in structure. The membrane was impregnated on a polyester cloth support. The HYPAN membrane in this case had greater than 75 mole % of acrylonitrite groups, the remainder being glutarimide-derived groups. The pores on this membrane were in the range of .02-1 micrometer. The test results were similar to those in Example 15.

### Example 21

The membrane of Example 20 was tried in a pseudo-liquid form as described in Example 17. Again similar successful results were obtained.

## Claims

1. A device for obtaining plasma from whole blood and determining blood analytes thereof, which comprises:
(a) a microporous plasma separation membrane (7) having a first surface and a second surface;
(b) a physical transport medium (5) contacted in part with said first surface of said microporous plasma separation membrane (7) which medium (5) is capable of moving at least a portion of blood to be tested from a remote location on said physical transport medium (5) to said first surface of said microporous plasma separation membrane (7); and
(c) a plasma collecting test membrane (11) in contact with the second surface of said microporous plasma separation membrane (7) for receiving said separated plasma, said test membrane (11) being capable of reacting with said plasma to display at least one analyte characteristic thereof;
characterised in that said microporous plasma separation membrane (7) is a skinned membrane, in which one or both surfaces of the skinned membrane (7) have smaller and uniform pore-sizes, as compared to the middle region of the skinned membrane (7).

2. The device of claim 1 wherein said skinned microporous plasma separation membrane (7) is cellulosic or is of nitrocellulose.

3. The device of claim 1 or claim 2 wherein said skinned microporous plasma separation membrane (7) has a nominal pore size of about 0.02 to about 10 micrometers.

4. The device of claim 3 wherein said skinned microporous plasma separation membrane (7) has a nominal pore size of about 1 to 5 micrometers.

5. The device of any of claims 1 to 4 wherein said physical transport medium (5) relies, at least in part, on capillary forces to move the blood.

6. The device of any of claims 1 to 5 wherein said physical transport medium (5) is a transport membrane sheet.

7. The device of claim 6 wherein said transport membrane sheet (5) is a sheet of fabric selected from woven fabric, non-woven fabric, gauze and monofilament yarn.

8. The device of any of claims 1 to 5 wherein said physical transport medium (5) constitutes grooves or channels (19) formed on an inert substrate (17) to promote movement of blood.

9. The device of any of claims 1 to 5 wherein said physical transport medium (5) is an open-structured mesh with openings of at least 1 micrometer in size and having hydrophilic surfaces.

10. The device of any of claims 1 to 9 further comprising:
(d) an absorbent storage medium (25) connected to said physical transport medium (5) wherein said physical transport medium (5) is capable of moving excess blood to said absorbent storage medium (25).

11. The device of any of claims 1 to 10 wherein said plasma collecting test membrane (11) includes one or more reactants which react with said plasma to effect colorimetric reactions of one or more analytes.

12. The device of any of claims 1 to 11 further including at least one inert substrate (3) to which at least one of said microporous plasma separation membrane (7), said plasma collecting test membrane (11) and said physical transport medium (5) is connected.

13. The device of any of claims 1 to 12 wherein a portion of said physical transport medium (5) with which the microporous plasma separation membrane (7) is not in contact, is exposed for applying a sample of blood thereto.

14. The device of any of claims 10 to 13 which further comprises:
(e) a top sheet (13) containing at least one aperture (15) through which a blood sample may be applied onto the physical transport medium (5), and at least a portion of this top sheet (13) overlying said plasma collecting test membranes (11), said portion having transparent areas to view one or more colorimetric reactions.

15. The device of claim 14 wherein portions of said transparent areas on the top sheet (13), have holes or apertures to ensure oxygen transport to the plasma collecting test membrane for reactions that utilize oxygen.

16. The device of claim 15 wherein said holes or apertures of oxygen transport are covered by transparent oxygen permeable membranes.

17. A method of obtaining plasma from whole blood and determining blood analytes thereof, which comprises applying a sample of blood to the first surface of the skinned microporous plasma separation membrane of a device according to any of claims 1 to 16 via said physical transport medium.

18. The method of claim 17 wherein said analyte characteristic is a colorimetric reaction result.

19. The method of claim 17 wherein said analyte characteristic is analyte concentration of one or more analytes selected from cholesterol, HDL cholesterol, triglycerides and glucose.

## Patentansprüche

1. Vorrichtung zur Gewinnung von Plasma aus dem vollständigen Blut und zur Bestimmung von dessen Blutanalyten, welche folgende Elemente aufweist:
(a) eine mikroporöse Plasma-Trennmembran (7), die eine erste Oberfläche und eine zweite Oberfläche hat;
(b) ein stoffliches Transportmittel (5), das teilweise mit der ersten Oberfläche der mikroporösen Plasma-Trennmembran (7) in Kontakt ist, wobei das Mittel (5) in der Lage ist, wenigstens einen Teil des zu prüfenden Blutes von einer entfernten Stelle auf dem stofflichen Transportmittel (5) zu der ersten Oberfläche der mikroporösen Plasma-Trennmembran (7) zu bewegen, und
(c) eine Plasmasammel-Prüfmembran (11) in Kontakt mit der zweiten Oberfläche der mikroporösen Plasma-Trennmembran (7) zur Aufnahme des abgetrennten Plasmas, wobei die Prüfmembran (11) in der Lage ist, mit dem Plasma zu reagieren, um wenigstens eines von dessen Analytcharakteristika anzuzeigen:
dadurch gekennzeichnet, daß die mikroporöse Plasma-Trennmembran (7) eine Skin-Membran ist, bei der eine oder beide Oberflächen der Skin-Membran (7) eine kleinere und einheitliche Porengröße als der Mittelbereich der Skin-Membran (7) haben.

2. Vorrichtung nach Anspruch 1, bei der die mikroporöse Plasma-Trenn-Skin-Membran (7) ein Celluloseerzeugnis oder aus Cellulosenitrat ist.

3. Vorrichtung nach Anspruch 1 oder Anspruch 2, bei der die mikroporöse Plasma-Trenn-Skin-Membran (7) eine nominelle Porengröße von etwa 0.02 bis etwa 10 »m hat.

4. Vorrichtung nach Anspruch 3, bei der die mikroporöse Plasma-Trenn-Skin-Membran (7) eine nominelle Porengröße von etwa 1 bis 5 »m hat.

5. Vorrichtung nach einem der Ansprüche 1 bis 4, bei der sich das stoffliche Transportmittel (5) zumindest teilweise auf die Kapillarwirkung stützt, um das Blut zu bewegen.

6. Vorrichtung nach einem der Ansprüche 1 bis 5, bei der das stoffliche Transportmittel (5) eine Transportmembranlage ist.

7. Vorrichtung nach Anspruch 6, bei der die Transportmembranlage (5) eine Lage eines textilen Flächengebildes ist, das aus Gewebe, Vliesstoff, Mull und Monofilseide ausgewählt wird.

8. Vorrichtung nach einem der Ansprüche 1 bis 5, bei der das stoffliche Transportmittel (5) Rillen oder Kanäle (19) bildet, die auf einem inerten Substrat (17) geformt werden, um die Bewegung des Blutes zu fördern.

9. Vorrichtung nach einem der Ansprüche 1 bis 5, bei der das stoffliche Transportmittel (5) ein offen strukturiertes Maschennetz mit Öffnungen von wenigstens 1 »m Größe ist und hydrophile Oberflächen hat.

10. Vorrichtung nach einem der Ansprüche 1 bis 9, die außerdem aufweist:
(d) ein absorbierendes Speichermittel (25), das mit dem stofflichen Transportmittel (5) verbunden ist, worin das stoffliche Transportmittel (5) in der Lage ist, überschüssiges Blut zu diesem absorbierenden Speichermittel (25) zu bewegen.

11. Vorrichtung nach einem der Ansprüche 1 bis 10, bei der die Plasmasammel-Prüfmembran (11) einen oder mehrere reagierende Stoffe einschließt, die mit dem Plasma reagieren, um kolorimetrische Reaktionen eines oder mehrerer Analyte zu bewirken.

12. Vorrichtung nach einem der Ansprüche 1 bis 11, die außerdem wenigstens ein inertes Substrat (3) einschließt, mit dem wenigstens eines der Elemente mikroporöse Plasma-Trennmembran (7), Plasmasammel-Prüfmembran (11) und stoffliches Transportmittel (5) verbunden ist.

13. Vorrichtung nach einem der Ansprüche 1 bis 12, bei der ein Teil des stofflichen Transportmittels (5), der nicht mit der mikroporösen Plasma-Trennmembran (7) in Kontakt ist, exponiert ist, um eine Probe des Blutes auf diesen aufzubringen.

14. Vorrichtung nach einem der Ansprüche 10 bis 13, die außerdem aufweist:
(e) eine obere Lage (13), die wenigstens eine Öffnung (15) enthält, durch die eine Blutprobe auf das stoffliche Transportmittel (5) aufgebracht werden kann, und wobei wenigstens ein Teil dieser oberen Lage (13) die Plasmasammel-Prüfmembranen (11) überlagert, wobei dieser Teil transparente Zonen hat, um eine oder mehrere kolorimetrische Reaktionen zu sehen.

15. Vorrichtung nach Anspruch 14, bei der Teile der transparenten Zonen auf der oberen Lage (13) Löcher oder Öffnungen haben, um für Reaktionen. die Sauerstoff nutzen, den Sauerstofftransport zur Plasmasammel-Prüfmembran zu gewährleisten.

16. Vorrichtung nach Anspruch 15, bei der die Löcher oder Öffnungen für den Sauerstofftransport durch transparente, sauerstoffdurchlässige Membranen bedeckt sind.

17. Verfahren zur Gewinnung von Plasma aus dem vollständigen Blut und zur Bestimmung von dessen Blutanalyten, welches das Aufbringen einer Blutprobe auf eine erste Oberfläche der mikroporösen Plasma-Trenn-Skin-Membran einer Vorrichtung nach einem der Ansprüche 1 bis 16 mittels des stofflichen Transportmittels umfaßt.

18. Verfahren nach Anspruch 17, bei dem das Analytcharakteristikum das Ergebnis einer kolorimetrischen Reaktion ist.

19. Verfahren nach Anspruch 17, bei dem das Analytcharakteristikum eine Analytkonzentration von einem oder mehreren Analyten ist, die aus Cholesterol, HDL-Cholesterol, Triglyceriden und Glucose ausgewählt werden.

## Revendications

1. Dispositif pour obtenir du plasma à partir de sang complet et pour déterminer les composantes de sang correspondantes, comprenant:
(a) une membrane microporeuse de séparation du plasma (7) comportant une première surface et une deuxième surface;
(b) un agent physique de transport (5), contactant en partie ladite première surface de ladite membrane microporeuse de séparation du plasma (7), cet agent (5) étant capable de déplacer au moins une partie du sang à analyser, d'un emplacement écarté sur ledit agent physique de transport (5) vers ladite première surface de ladite membrane microporeuse de séparation du plasma (7); et
(c) une membrane d'essai de collecte du plasma (11), en contact avec la deuxième surface de ladite membrane microporeuse de séparation du plasma (7), pour recevoir ledit plasma séparé, ladite membrane d'essai (11) pouvant réagir avec ledit plasma pour révéler au moins une caractéristique d'une composante correspondante;
caractérisé en ce que ladite membrane microporeuse de séparation du plasma (7) est une membrane à pellicule, une surface ou les deux surfaces de la membrane à pellicule (7) ayant des grosseurs de pores plus petites et uniformes, en comparaison avec la région médiane de la membrane à pellicule (7).

2. Dispositif selon la revendication 1, dans lequel ladite membrane à pellicule microporeuse de séparation du plasma (7) est cellulosique ou est composée de nitrocellulose.

3. Dispositif selon les revendications 1 ou 2, dans lequel ladite membrane à pellicule microporeuse de séparation du plasma (7) a une grosseur nominale des pores comprise entre environ 0.02 et environ 10 micromètres.

4. Dispositif selon la revendication 3, dans lequel ladite membrane à pellicule microporeuse de séparation du plasma (7) a une grosseur nominale des pores comprise entre environ 1 et 5 micromètres.

5. Dispositif selon l'une quelconque des revendications 1 à 4, dans lequel ledit agent physique de transport (5) se base, du moins en partie, sur des forces capillaires pour déplacer le sang.

6. Dispositif selon l'une quelconque des revendications 1 à 5, dans lequel ledit agent physique de transport (5) est une feuille de membrane de transport.

7. Dispositif selon la revendication 6, dans lequel ladite feuille de membrane de transport (5) est une feuille de tissu sélectionnée parmi du tissu tissé, du tissu non tissé, de la gaze et du fil monofilament.

8. Dispositif selon l'une quelconque des revendications 1 à 5, dans lequel ledit agent physique de transport (5) constitue des rainures ou des cannelures (19) formées sur un substrat inerte (17) pour faciliter le déplacement du sang.

9. Dispositif selon l'une quelconque des revendications 1 à 5, dans lequel ledit agent physique de transport (5) est un tamis à structure ouverte, comportant des ouvertures d'une grosseur d'au moins un micromètre et ayant des surfaces hydrophiles.

10. Dispositif selon l'une quelconque des revendications 1 à 9, comprenant en outre:
(d) un agent de stockage absorbant (25) connecté audit agent physique de transport (5), ledit agent physique de transport (5) pouvant déplacer le sang excédentaire vers ledit agent de stockage absorbant (25).

11. Dispositif selon l'une quelconque des revendications 1 à 10, dans lequel ladite membrane d'essai de collecte du plasma (11) englobe un ou plusieurs réactifs réagissant avec ledit plasma pour entraîner des réactions colorimétriques d'une ou de plusieurs composantes.

12. Dispositif selon l'une quelconque des revendications 1 à 11, englobant en outre au moins un substrat inerte (3), auquel sont connectés au moins une desdites membranes microporeuses de séparation du plasma (7), desdites membranes d'essai de collecte du plasma (11) et desdits agents physiques de transport (5).

13. Dispositif selon l'une quelconque des revendications 1 à 12, dans lequel une partie dudit agent physique de transport (5), non contactée par ladite membrane microporeuse de séparation du plasma (7), est exposée en vue d'y appliquer un échantillon de sang.

14. Dispositif selon l'une quelconque des revendications 10 à 13, comprenant en outre:
(e) une feuille supérieure (13) contenant au moins une ouverture (15), à travers laquelle un échantillon de sang peut être appliqué sur l'agent physique de transport (5), et au moins une partie de cette feuille supérieure (13) étant superposée auxdites membranes d'essai de collecte du plasma (11), ladite partie comportant des zones transparentes permettant de voir une ou plusieurs réactions colorimétriques.

15. Dispositif selon la revendication 14, dans lequel des parties desdites zones transparentes sur la feuille supérieure (13) comportent des trous ou des ouvertures, pour assurer le transport d'oxygène vers la membrane d'essai de collecte du plasma, pour les réactions utilisant de l'oxygène.

16. Dispositif selon la revendication 15, dans lequel lesdits trous ou lesdites ouvertures de transport d'oxygène sont couverts par une membrane transparente perméable à l'oxygène.

17. Procédé pour obtenir du plasma à partir de sang complet et pour déterminer les composantes de sang correspondantes, comprenant l'application d'un échantillon de sang sur la première surface de la membrane microporeuse à pellicule de séparation du plasma d'un dispositif selon l'une quelconque des revendications 1 à 16, par l'intermédiaire dudit agent physique de transport.

18. Procédé selon la revendication 17, dans lequel ladite caractéristique de la composante est un résultat d'une réaction colorimétrique.

19. Procédé selon la revendication 17, dans lequel ladite caractéristique de la composante est la concentration analytique d'une ou de plusieurs composantes sélectionnées parmi le cholestérol, le cholestérol HDL, les triglycérides et le glucose.
